# EUROPEAN PATENT SPECIFICATION

(11) **EP 3 762 716 B1**
(45) Date of publication and mention of the grant of the patent: **06.05.2026**
(21) Application number: 19764583.1
(22) Date of filing: 08.03.2019
(51) Int. Cl.: B01D 15/38, B01J 20/285, B01J 20/289, C08B 37/00, C08H 1/00, C08L 5/12, C08L 89/00

(54) **A PROTEIN-BINDING PRODUCT, A DEVICE CONTANING SAID PROTEIN-BINDING PRODUCT AND A METHOD FOR EXTRACORPOREAL REDUCTION OF THE LEVEL OF PROTEIN IN BLOOD PLASMA**
PROTEINBINDENDES PRODUKT, DAS PROTEINBINDENDE PRODUKT ENTHALTENDE VORRICHTUNG UND VERFAHREN ZUR EXTRAKORPORALEN REDUKTION DER PROTEINKONZENTRATION IM BLUTPLASMA
PRODUIT DE LIAISON PROTÉIQUE, DISPOSITIF CONTENANT LEDIT PRODUIT DE LIAISON PROTÉIQUE ET PROCÉDÉ DE RÉDUCTION EXTRACORPORELLE DU NIVEAU DE PROTÉINE DANS LE PLASMA SANGUIN

(30) Priority: 08.03.2018 SE 1800056
(43) Date of publication of application: 13.01.2021
(73) Proprietor: Glycorex Transplantation AB, 223 70 Lund (SE)
(72) Inventor: NILSSON, Kurt, 239 42 FALSTERBO (SE)
(74) Representative: AWA Sweden AB
(86) International application number: PCT/SE2019/050211
(87) International publication number: WO 2019/172836

(56) References cited:
- WO-A1-2013/062479
- WO-A1-2015/099826
- US-A- 6 084 072
- US-A1- 2002 146 814
- US-A1- 2007 059 769
- US-A1- 2007 269 833
- WALSER P J ET AL: "Ligand interactions of the Coprinopsis cinerea galectins", FUNGAL GENETICS AND BIOLOGY, SAN DIEGO, CA, US, vol. 42, no. 4, 1 April 2005 (2005-04-01), pages 293 - 305, XP027233753, ISSN: 1087-1845, [retrieved on 20050303]
- PUROHIT, S. ET AL.: "Multiplex glycan bead array for high throughput and high content analyses of glycan binding proteins", NATURE COMMUNICATIONS, vol. 258, no. 9, December 2018 (2018-12-01), pages 1 - 12, XP055636225
- AHMAD, N. ET AL.: "Thermodynamic binding studies of cell surface carbohydrate epitopes to galectins-1, -3, and -7: Evidence for differential binding specificities", CANADIAN. JOURNAL OF. CHEMISTRY, vol. 80, no. 8, 2002, pages 1096 - 1104, XP055636232, Retrieved from the Internet <URL:https://www.nrcresearchpress.com/doi/pdfplus/10.1139/v02-162>
- CARLSSON, S. ET AL.: "Intracellular sorting of galectin-8 based on carbohydrate fine specificity", GLYCOBIOLOGY, vol. 17, no. 9, 22 June 2007 (2007-06-22) - September 2007 (2007-09-01), pages 906 - 912, XP055636234

## Description

### Technical Field of the Invention

The present invention relates to a protein-binding product comprising porous polymer beads, a device containing said protein-binding product and a method for extracorporeal elimination of at least one galactose-binding protein and optionally at least one other protein in blood plasma.

### Background

An example of galactose binding proteins in blood plasma is a group of proteins called galectins. Galectins are a class of proteins that bind specifically to β-galactoside sugars. These proteins have been associated with a range of diseases. E. g. increased levels of galectins in blood have been detected in humans suffering from inflammatory conditions, autoimmune diseases and cancer.

A range of galactoside derivatives, e. g. galactose compounds modified with aliphatic and or aromatic compounds, have been proposed as injectable compounds which potentially can inhibit the galectin activity.

Walser P J et al (2005) "Ligand interactions of the Coprinopsis cinerea galectins", Fungal Genetics and Biology, page 293-305, discloses ligand interactions of the galectins of a mushroom, *Coprinopsis cinerea.*

US2002/146814 relates to an apparatus for containing a biologically active saccharide covalently bound by at least one spacer to a cross linked matrix.

WO2013/062479 relates to a method for extracorporeal elimination of components in whole blood or blood plasma in a treatment device containing an adsorbent. The adsorbent comprise a matrix to which a ligand is covalently bound.

However, since galectins are important in the normal cell and tissue activities, the injection of the galactoside derivatives may lead to unfavourable side effects. A long-term use of these drugs might lead to more or less toxic effects. It is also difficult to assess the concentration of these injected or otherwise administered organic compounds in the different body parts, and thus to ascertain the inhibition effect achieved at the desired sites of galectin action.

### Summary of the Invention

An object of the present invention is to overcome the drawbacks and disadvantages disclosed above. This object is achieved by providing a protein-binding product comprising porous polymer beads, a device containing said protein-binding product and a method for extracorporeal elimination of at least one galactose-binding protein and optionally at least one other protein in blood plasma.

The present invention refers to a product as defined in independent claim 1, more precisely to a protein-binding product comprising one or more porous polymer beads, wherein at least one ligand is bound to the surfaces of said polymer beads via a spacer (R), and wherein said at least one ligand is a derivative of Galβ1-3GlcNAcβ-O-, wherein the 3-OH group of Gal is substituted with one of the following groups:
a) a 3-OR2 group where the R2 group contains an alkyl group or an aromatic group,
b) a 3-OR2 group where the R2 group contains a D-galactosyl group or GalNAc group,
c) a 3-NH-CO-R2 group where the R2 group contains an alkyl group or an aromatic group,
d) a 3,5-dimethoxybenzamido group, and/or

wherein said at least one ligand is Galβ1-3GalNAcβ-O-and/or a derivative thereof, wherein said derivative of said at least one ligand has a structure in which the 3-OH group of Gal is substituted with one of the groups a)-d) above, and/or
wherein said at least one ligand is a derivative of Galβ1-4GlcNAcβ-O-, wherein the 3-OH group of Gal is substituted with one of the groups a)-d) above, except for b) where the R2 group contains a D-galactosyl group,
wherein said at least one ligand has the ability to bind at least one galectin in human blood, and wherein said protein-binding product in addition comprises polymer beads having at least one covalently bound compound or ligand having the ability to bind at least one antibody in human blood.

The present invention refers also to a method as defined in independent claim 13, more precisely to a method for extracorporeal reduction of the level of at least one galectin and at least one antibody in blood plasma, said method comprising the steps of:
a) passing the blood plasma through the device according to claim 12,
b) allowing said at least one galactin, and said at least one antibody, preferably a blood group A specific antibody, a blood group B specific antibody, an autoantibody, more preferably an AChR (acetylcholine receptor) antibody and a MuSK (muscle-specific kinase) receptor antibody; to bind to said at least one antibody-binding-ligand,
c) allowing at least one blood plasma volume to pass through the device after passage of one blood plasma volume through the device,
d) repeating step c) until the desired reduction of the level of said at least one galectin and said at least one antibody in the blood plasma is achieved.

Disclosed but not claimed is also a method, more precisely a method for extracorporeal reduction of the level of at least one galactose-binding protein and optionally at least one other protein in blood plasma, said method comprising the steps of:
a) passing the blood plasma through the device according to claim 12,
b) allowing said at least one galactose-binding protein, preferably a galectin, and optionally at least one other protein, preferably an antibody, more preferably a blood group A specific antibody, a blood group B specific antibody, an autoantibody, more preferably an AChR (acetylcholine receptor) antibody and a MuSK (muscle-specific kinase) receptor antibody, or a toxin to bind to said at least one ligand of the protein binding product,
c) allowing at least one blood plasma volume to pass through the device after passage of one blood plasma volume through the device,
d) repeating step c) until the desired reduction of the level of said at least one galactose-binding protein and optionally at least one other protein in the blood plasma is achieved,
e) optionally performing dialysis concomitantly with the steps above,
f) continuously returning the treated blood plasma to the patient.

Further embodiments of the present invention are disclosed in the subsequent independent claims.

In another aspect, the present invention also refers to a to use of said protein-binding product for reduction of the levels of said at least one galectin, preferably galectin-1, galectin-3 and galectin-8; and the levels of said at least one antibody, preferably a blood group A specific antibody, a blood group B specific antibody, an autoantibody, more preferably an AChR (acetylcholine receptor) antibody and a MuSK (muscle-specific kinase) receptor antibody, in human blood plasma.

In further aspect, the present invention also refers to a device containing said protein-binding product.

### Detailed Description of the Present Invention and Preferred Embodiments Thereof

With the present invention the treatment method of a patient in need for reduction of the levels of galectins in the blood can be adjusted for each patient in order to achieve the desired treatment effect, or a desired galectin level, by determining the reduction of the levels of galectins in a blood sample of the patient.

Throughout the application text, the expression "blood" is generally intended to mean whole blood, but here it also covers partially purified blood components, e.g. IVIG (intravenous IG), unless otherwise is stated. The expression "blood plasma" used throughout the application text is intended to also cover partly purified plasma, unless otherwise is stated. The expression "the surfaces of the polymer beads" is intended to cover both the outer surface of the polymer beads and the surfaces in the pores of the polymer beads."

In the present invention porous polymer is provided, wherein the polymer is a non-soluble beaded matrix, which contains at least one covalently bound ligand, that is bound via a spacer (R) to the matrix and where the ligand is at least one of the following: a derivative of Galβ1-3GlcNAcβ-O-, wherein the 3-OH group of Gal is substituted with one of the following groups:
a) a 3-OR2 group where the R2 group contains an alkyl group or an aromatic group,
b) a 3-OR2 group where the R2 group contains a D-galactosyl group or GalNAc group,
c) a 3-NH-CO-R2 group where the R2 group contains an alkyl group or an aromatic group,
d) a 3,5-dimethoxybenzamido group, and/or

wherein said at least one ligand is Galβ1-3GalNAcβ-O-and/or a derivative thereof, wherein said derivative of said at least one ligand has a structure in which the 3-OH group of Gal is substituted with one of the groups a)-d) above, and/or
wherein said at least one ligand is a derivative of Galβ1-4GlcNAcβ-O-, wherein the 3-OH group of Gal is substituted with one of the groups a)-d) above, except for b) where the R2 group contains a D-galactosyl group,

The spacer, also called R, consists of or comprises an aliphatic and/or aromatic compound, which is glycosidically linked or bound to at least one of the following ligand compounds: a derivative of Galβ1-3GlcNAcβ-O- wherein the 3-OH group of Gal is substituted with one of the following groups:
a) a 3-OR2 group where the R2 group contains an alkyl group or an aromatic group,
b) a 3-OR2 group where the R2 group contains a D-galactosyl group or GalNAc group,
c) a 3-NH-CO-R2 group where the R2 group contains an alkyl group or an aromatic group,
d) a 3,5-dimethoxybenzamido group, and/or

wherein said at least one ligand is Galβ1-3GalNAcβ-O-and/or a derivative thereof, wherein said derivative of said at least one ligand has a structure in which the 3-OH group of Gal is substituted with one of the groups a)-d) above, and/or
wherein said at least one ligand is a derivative of Galβ1-4GlcNAcβ-O-, wherein the 3-OH group of Gal is substituted with one of the groups a)-d) above, except for b) where the R2 group contains a D-galactosyl group.. Examples of R are (CH₂)ₙNH-, (CH₂)ₙPhNH-, PhNH- and Ph(CH₂)ₙNH-, wherein Ph is a phenyl group and n is an integer, preferably wherein n is one of 1, 2, 3, 4, 5 or 6. The spacer is covalently linked to the polymer, more precisely to the surfaces of the polymer beads, i. e. both to the outer surface of the polymer beads and to the polymer surfaces in the pores of the polymer beads. As the polymer is porous, this allows entrance of plasma containing galactose binding proteins and larger molecules.

In a preferred embodiment of the invention the polymer is agarose or crosslinked agarose beads, hydroxymethacrylate beads or some other kinds of porous plastic beads. The spacer is covalently bound directly to carbon atoms of the polymer or to a second spacer covalently bound to the polymer.

Thus, as an example, the NH group of the spacer (R) can bind covalently to carbon atoms of a portion of the galactose units of e. g. agarose beads after so-called activation of a portion of the galactose residues in agarose with tosyl chloride (pyridine used as catalyst in e. g. acetone). This type of activation results in that tosyl groups are introduced on a portion of the galactose-OH groups of the agarose beads. Tosyl groups are released upon reaction of the tosylated agarose with amino groups to give a -NH-CH-galactose (in agarose) linkage, which is highly stable.

The binding to the polymer of the above/mentioned ligand can also be achieved via a second spacer by e. g. first covalently coupling of an aliphatic and/or aromatic compound containing e. g. a carboxyl group to e. g. galactose residues of the cross-linked agarose beads mentioned above. In another example, activation of the carboxyl group of the second spacer to give NHS-CO-second spacer-agarose (NHS is N-hydroxysuccinimide) allows for amide linkage formation (NH-CO) between the first and the second spacer, which is also a stable covalent linkage.

The Gal unit in the ligands defined above denotes a D-galactosyl group. HexNAc in the Galβ1-3HexNAcβ-O- ligand above is chosen from GIcNAc or GalNAc (N-acetyl-D-glucosamine or N-acetyl-D-galactosamine). As to the derivative of the Galβ1-3HexNAcβ-O- and Galβ1-4GlcNAcβ-O-, ligands, the derivative has the structure in which the 3-OH group of the Gal unit in any of the above with an α2-3-linked sialyl group, an α2-6-linked sialyl group, a β1-3-linked GlcNAc, an α1-3-linked D-galactosyl group or an α1-3-linked GalNAc group. In the two latter examples the 2-OH group of Gal in Gal β1-3HexNAcβ-O- and Galβ1-4GlcNAcβ-O-, may be additionally substituted with e. g. an α1-2-linked L-fucosyl group. As another example, the 3-OH group of Gal in Galβ1-3HexNAcβ-O- or Galβ1-4GlcNAcβ-O- may be substituted with a group containing an aromatic group.

An alternative way to define the structure of said at least one ligand is that it has a structure, wherein at least the 3-OH group of Gal is substituted with one of the following groups:
a 3-O-R2 group where the R2 group contains an alkyl group or an aromatic group,
a 3-NH-O-R2 group where the R2 group contains a sialyl group or a D-galactosyl group or GalNac group,
a 3-O-R2 group where the R2 group contains an alkyl group or an aromatic group,
a 3,5-dimethoxybenzamido group.

The above choice of the ligand, Galβ1-3HexNAcβ-O- or Galβ1-4GlcNAcβ-O- or a derivative thereof, as described above, will be determined by the β-galactoside-binding protein or β-galactoside-binding proteins whose levels are desired to be reduced. Thus, e. g. a Galβ1-4GlcNAc β-O- ligand or a derivative thereof is preferred when the level of e. g. galectin-3 is desired to be reduced, whereas e. g. α2-3-sialylated Galβ1-3GalNAcβ-O- or a derivative thereof is preferred for the reduction of the level of galectin-8, and α2-6-sialylated Galβ1-3GlcNAcβ- or a derivative thereof is preferred for the reduction of the level of galectin-1.

At least 5 µmol of one or more of the following ligands Galβ1-3HexNAcβ-O- and Galβ1-4GlcNAcβ-O- per mL polymer is used, preferably 5-20 µmol of ligand per mL polymer. When a derivative of any of the ligands Galβ1-3HexNAcβ-O- and Galβ1-4GlcNAcβ-O- is used according to the invention described above, a lower concentration of the ligand can be used per mL of matrix, e. g. 0.1-5 µmol ligand per mL polymer.

The polymer with the ligand bound thereto according to the present invention can be used to quantitatively reduce the level of galectin in human blood plasma. Thus, the level of galectin-3 in blood plasma from a patient can be reduced by more than 50 % in one single passage through a column filled with porous polymer beads provided with a covalently bound ligand via a spacer, wherein the ligand e. g. is Galβ1-4GlcNAcβ-O, which can be modified in Gal as described above with a fucosyl group and with either GalNAc or Gal.

According to the invention, the reduction of the level of galectin, i. e. the treatment effect, will increase similarly for each patient plasma plasma volume treated, i. e. for each blood plasma volume that has passed the column when used in a continuous extracorporeal treatment with a column connected via a tubing to a blood plasma separator which in turn is connected via tubings to the patient. The blood plasma that has passed the column is in one embodiment returned to the blood line after passing through the blood plasma separator and thus continuously returned to the patient. In another embodiment the plasma which has passed the column is collected, i. e. is not returned to the patient. Therefore, in the example above, after the passage of the volume of blood plasma e. g. corresponding to the volume of blood plasma in 4 patients through the column, more than 90 % of the original level of galectin in the plasma is removed by being bound to the column. According to the present invention, the volume of polymer in the column is e. g. 60 mL or between 5 and 70 mL. A higher or a lower volume can be chosen depending on the used flow rate of the blood plasma and the plasma volume of the patient. The flow rate is chosen preferably in a range of between 10 and 60 mL per minute. The reduction of the level of galectin in the patient plasma can be lower due to unintended equilibration with extravascular galectin. If this happens, the treatment could be prolonged, i. e. more patient plasma volumes may be treated per treatment session.

When the column contains a polymer-spacer-ligand complex, i. e. a protein-binding product, according to the present invention, e. g. a disaccharide derivative in which the 3-OH group of Gal in any of the disaccharide structures disclosed above is substituted with a 3-NH-CO-R2 group or with a 3-O-R2 group as described above, a higher reduction of the level of galectin per treated patient blood plasma volume can be achieved. For this type of polymer, a lower amount of polymer can be chosen in the column, i. e. amount of polymer in the lower range of the range mentioned above. The polymer according to the invention is not saturated with bound galectin during prolonged treatments. The reason for that is that the molar quantity of the carbohydrate or carbohydrate derivative constituting the ligand is higher than the molar quantity of galectin in the blood of the patient. This high reduction is surprising in view of what is known, since human blood plasma contains a range of glycoproteins that normally bind to galectin-3 and could thus be expected to inhibit the binding of the galectin to the polymer beads provided with the ligands.

In another embodiment, the device according to the invention contains, in addition to the above described polymer with a covalently bound carbohydrate or carbohydrate derivative as ligand, a polymer with at least one other covalently bound compound or ligand than that mentioned above, which can bind a protein other than a galectin, e. g. an antibody, a protein or a toxin. A simultaneous reduction of the levels of galectin, antibody, toxin or said other protein can thus be achieved in human blood plasma with the treatment method according to the present invention. The total volume of the two polymers in the column is preferably of a value presented above, and the volume ratio of the two polymers is preferably in the range between 1/0.1-0.1/1.

An example of such an antibody is a blood group specific antibody, such as a blood group A or B specific antibody, or an antibody involved in an autoimmune disease, preferably an autoantibody involved in Myasthenia gravis, more preferably an autoantibody for the antigens in an AChR (acetylcholine receptor) or a MuSK (muscle-specific kinase) receptor, or an autoantibody to an antigen involved in another autoimmune disease. The antigen is covalently bound to the type of polymer described above and using the same activation methods as described above. In the case of covalently bound antigen used for binding to the autoantibody, the amount of bound antigen is chosen to be between 0.1 to 5 mg antigen per mL polymer. In this embodiment a reduction of the levels of both galactose-binding proteins, such as galectins, and another protein, such as an antibody or a toxin, in human blood can be achieved.

The simultaneous reduction of the levels of a blood group specific antibody and a galectin achieved by the present invention is described below. The polymer beads used in this case are made of one polymer with e. g. covalently bound blood group A and/or B antigen at a concentration in a range of 1 to 4 µmol per mL polymer, and another polymer with a covalently bound galectin-binding ligand at the concentration described above. In this way, the levels of both blood group A and/or B specific antibody can be reduced, as well as the level of galectin in human blood. This embodiment can e. g. be of interest in a transplant setting for treatment of the recipient where the donor and recipient are blood group incompatible and where the recipient has increased galectin levels. Increased galectin levels have been shown to be a marker for e. g. kidney and liver fibrosis. Here the volume of the polymer provided with the galectin-binding ligand is lower than the volume of the polymer provided with the blood group specific antibody-binding ligand. If no simultaneous reduction of the levels of blood group specific antibody and galectin is desired, e. g. in another application than a blood group incompatible transplantation, a galectin-binding ligand which does not bind anti-B antibody in blood group A plasma, i. e. for reduction of the level of galectin in a patient with the blood group A, or which does not bind anti-A antibody in blood group B plasma, i. e. for reduction of the level of galectin in a patient with the blood group B, can be used.

The treatment method can be performed in a continuous fashion in an extracorporeal system, where the blood is continuously pumped from a reservoir or a patient to a plasma separator, which continuously separates out blood plasma from the blood line. The blood plasma is continuously treated with the device according to the invention, and the treated plasma with a reduced galectin content, optionally also with a reduced content of e. g. blood group antibody, autoantibody, other protein, or toxin, as described above, is continuously returned to the blood line and circulated back to the reservoir or to the patient. In an alternative plasma treatment method, the treated plasma is collected in a blood bag.

The polymer beads provided with ligands according to the invention can e. g. be filled in a column and be used in a continuous extracorporeal treatment method.

As mentioned above, several patient plasma volumes can be treated in a single treatment since the polymer beads provided with ligands will not be saturated with e. g. galectin and, optionally blood group antibody, autoantibody or other protein, since the molar quantity of the ligand used is higher than that of the compound(s) removed from the plasma.

In another embodiment of the present invention the device is a column. The column inlet is connected to a plasma filter or separator, which in turn is connected to the blood line out from the patient. The plasma filter continuously filters out plasma from the blood line. The blood plasma exiting the plasma filter passes through a tubing and then through the column with the above-described polymer beads. The treated plasma from the column outlet is returned via a tubing to the blood line, which is continuously returned back to the patient with the extracorporeal treatment continuous flow. The blood line back to the patient contains a reduced amount of galectin and optionally a reduced amount of any other protein, such as an antibody or a toxin.

The polymer beads can in another embodiment be filled in a tubing connected to the inlet and the outlet of a plasma filter, which is connected to the blood line from a patient and to the blood line back to patient or a blood reservoir. The polymer beads filled into the tubing are circulated by a pump through the plasma filter in the direction opposite to the flow of the blood line and within the space located between the porous hollow fibres present inside the plasma filter. Blood is continuously pumped from the patient to the plasma filter through the hollow fibres and back to the patient. Blood plasma is then filtered out through the hollow fibre pores and into the space between the hollow fibres and out into the connected tubing. The blood plasma is then continuously pumped through the tubing back to the space between the hollow fibres. The flow rate of the blood plasma line is higher than the flow of the blood line which is reverse to the one which is used in the above-mentioned column treatment. The so-treated blood plasma with a reduced galectin level is continuously filtered through the hollow fibre pores back to the blood line in the hollow fibres. The returned blood line to the patient or the reservoir contains a reduced amount of β-galactose binding protein and optionally also a reduced level of any other protein. In this way the level of galectin(s) and optionally other proteins (antibody and/or toxin) is reduced in the blood plasma contained within the blood reservoir or the patient. In another embodiment, the above column treatment is performed simultaneously with dialysis. This embodiment is of interest in a dialysis treatment per se of a patient with increased levels of galectin, but also for treatment of blood group incompatible recipients who have increased levels of galectin. Here, a conventional dialysis is performed but with the dialysis machine blood line and after the passage through the dialysis filter connected to the plasma separator. The filtered out blood plasma passes through the column as described above, and the treated blood plasma is returned to the blood line in a continuous fashion. In this way both dialysis and a reduced level of galectin, optionally also of any other protein, in human blood plasma is achieved.

## Claims

1. A protein-binding product comprising one or more porous polymer beads, wherein at least one ligand is bound to the surfaces of said polymer beads via a spacer (R), and
wherein said at least one ligand is a derivative of Galβ1-3GlcNAcβ-O-,
wherein the 3-OH group of Gal is substituted with one of the following groups:
a) a 3-OR2 group where the R2 group contains an alkyl group or an aromatic group,
b) a 3-OR2 group where the R2 group contains a D-galactosyl group or GalNAc group,
c) a 3-NH-CO-R2 group where the R2 group contains an alkyl group or an aromatic group,
d) a 3,5-dimethoxybenzamido group, and/or
wherein said at least one ligand is Galβ1-3GalNAcβ-O-and/or a derivative thereof, wherein said derivative of said at least one ligand has a structure in which the 3-OH group of Gal is substituted with one of the groups a)-d) above, and/or
wherein said at least one ligand is a derivative of Galβ1-4GlcNAcβ-O-,
wherein the 3-OH group of Gal is substituted with one of the groups a)-d) above, except for b) where the R2 group contains a D-galactosyl group,
wherein said at least one ligand has the ability to bind at least one galectin in human blood, and wherein said protein-binding product in addition comprises polymer beads having at least one covalently bound compound or ligand having the ability to bind at least one antibody in human blood.

2. The protein-binding product according to claim 1, wherein said at least one antibody is a blood group A specific antibody, a blood group B specific antibody, or an autoantibody.

3. The protein-binding product according to claim 2, wherein said autoantibody is anAChR (acetylcholine receptor) antibody or a MuSK (muscle-specific kinase) receptor antibody.

4. The protein-binding product according to any one of the preceding claims, wherein said at least one ligand having the ability to specifically bind to galectins in human blood plasma preferably is galectin-1, galectin-3, and galectin-8.

5. The protein-binding product according to any one of claims 1 and 2, wherein said ligand having the ability to bind at least one antibody is a blood group A and/or B antigen.

6. The protein-binding product according to any one of the preceding claims, wherein the concentration of the ligand is in a range of 0.1-20 µmol per mL polymer, preferably 5-20 µmol per mL polymer.

7. The protein-binding product according to any one of the preceding claims, wherein the concentration of the at least one ligand is in a range of 0.1-5 µmol per mL polymer.

8. The protein-binding product according to any one of the preceding claims, wherein the spacer (R) is covalently bound to the surfaces of said porous polymer beads and is glycosidically bound to the ligand.

9. The protein-binding product according to any one of the preceding claims, wherein the spacer is chosen from the group consisting of (CH₂)ₙNH-, (CH₂)ₙPhNH-, PhNH- and Ph(CH₂)ₙNH-, wherein Ph is a phenyl group and n is an integer, preferably wherein n is one of 1, 2, 3, 4, 5 or 6.

10. The protein-binding product according to any one of the preceding claims, wherein said porous polymer beads are agarose or cross-linked agarose beads, hydroxymethacrylate beads or other porous plastic beads.

11. Use of said protein-binding product according to any one of claims 1-10 for extracorporeal reduction of the levels of said at least one galectin, preferably galectin-1, galectin-3 and galectin-8; and the levels of said at least one antibody, preferably a blood group A specific antibody, a blood group B specific antibody, an autoantibody, more preferably an AChR (acetylcholine receptor) antibody and a MuSK (muscle-specific kinase) receptor antibody, in human blood plasma.

12. A device containing said protein-binding product according to any one of claims 1-10, wherein said device is a column or a tubing.

13. A method for extracorporeal reduction of the level of at least one galectin and at least one antibody in human blood plasma, said method comprising the steps of:
a) passing the blood plasma through the device according to claim 12,
b) allowing said at least one galactin and said at least one antibody, preferably a blood group A specific antibody, a blood group B specific antibody, an autoantibody, more preferably an AChR (acetylcholine receptor) antibody and a MuSK (muscle-specific kinase) receptor antibody; to bind to said at least one antibody-binding-ligand,
c) allowing at least one blood plasma volume to pass through the device after passage of one blood plasma volume through the device,
d) repeating step c) until the desired reduction of the level of said at least one galectin and said at least one antibody in the blood plasma is achieved.

14. The method according to any of claims 13, wherein
- the derivative of Galβ1-4GlcNAcβ-O- or a derivative thereof according to claim 1 is used as ligand when the level of galectin-3 is to be reduced.

15. A method according to any of claims 13 and 14, wherein said method additionally comprises at least one of the following steps:
measuring the levels of said at least one galectin and/or said at least one antibody during the treatment and
calculating the reduction of the levels of galactose-binding proteins and/or at least one other protein.

## Patentansprüche

1. Proteinbindendes Produkt, umfassend eine oder mehrere poröse Polymerkügelchen, wobei mindestens ein Ligand über einen Spacer (R) an die Oberflächen der Polymerkügelchen gebunden ist und
wobei es sich bei dem mindestens einen Liganden um ein Derivat von Galβ1-3GlcNAcβ-O- handelt,
wobei die 3-OH-Gruppe von Gal durch eine der folgenden Gruppen substituiert ist:
a) eine 3-OR2-Gruppe, bei der die R2-Gruppe eine Alkylgruppe oder eine aromatische Gruppe enthält,
b) eine 3-OR2-Gruppe, bei der die R2-Gruppe eine D-Galactosylgruppe oder GalNAc-Gruppe enthält,
c) eine 3-NH-CO-R2-Gruppe, bei der die R2-Gruppe eine Alkylgruppe oder eine aromatische Gruppe enthält,
d) eine 3,5-Dimethoxybenzamidogruppe, und/oder
wobei es sich bei dem mindestens einen Liganden um Galβ1-3GalNAcβ-O- und/oder ein Derivat davon handelt, wobei das Derivat des mindestens einen Liganden eine Struktur aufweist, in der die 3-OH-Gruppe von Gal durch eine der obigen Gruppen a) bis d) substituiert ist, und/oder
wobei es sich bei dem mindestens einen Liganden um ein Derivat von Galβ1-4GlcNAcp-O- handelt,
wobei die 3-OH-Gruppe von Gal durch eine der obigen Gruppen a) bis d) substituiert ist, mit Ausnahme von b), bei der die R2-Gruppe eine D-Galactosylgruppe enthält,
wobei der mindestens eine Ligand die Fähigkeit aufweist, mindestens ein Galectin in menschlichem Blut zu binden, und wobei das proteinbindende Produkt darüber hinaus Polymerkügelchen mit mindestens einer kovalent gebundenen Verbindung bzw. einem kovalent gebundenen Liganden umfasst, die bzw. der die Fähigkeit aufweist, mindestens einen Antikörper in menschlichem Blut zu binden.

2. Proteinbindendes Produkt nach Anspruch 1, wobei es sich bei dem mindestens einen Antikörper um einen Blutgruppe-A-spezifischen Antikörper, einen Blutgruppe-B-spezifischen Antikörper oder einen Autoantikörper handelt.

3. Proteinbindendes Produkt nach Anspruch 2, wobei es sich bei dem Autoantikörper um einen AChR(Acetylcholin-Rezeptor)-Antikörper oder einen MuSK(muskelspezifische Kinase)-Rezeptor-Antikörper handelt.

4. Proteinbindendes Produkt nach einem der vorhergehenden Ansprüche, wobei es sich bei dem mindestens einen Ligand mit der Fähigkeit, an Galectine in menschlichem Blutplasma spezifisch zu binden, vorzugsweise um Galectin-1, Galectin-3 und Galectin-8 handelt.

5. Proteinbindendes Produkt nach einem der Ansprüche 1 und 2, wobei es sich bei dem Liganden mit der Fähigkeit, mindestens einen Antikörper zu binden, um ein Blutgruppe-A- und/oder -B-Antigen handelt.

6. Proteinbindendes Produkt nach einem der vorhergehenden Ansprüche, wobei die Konzentration des Liganden in einem Bereich von 0,1-20 µmol pro ml Polymer, vorzugsweise 5-20 µmol pro ml Polymer, liegt.

7. Proteinbindendes Produkt nach einem der vorhergehenden Ansprüche, wobei die Konzentration des mindestens einen Liganden in einem Bereich von 0,1-5 µmol pro ml Polymer liegt.

8. Proteinbindendes Produkt nach einem der vorhergehenden Ansprüche, wobei der Spacer (R) an die Oberflächen der porösen Polymerkügelchen kovalent und an den Liganden glycosidisch gebunden ist.

9. Proteinbindendes Produkt nach einem der vorhergehenden Ansprüche, wobei der Spacer aus der Gruppe bestehend aus (CH₂)ₙNH-, (CH₂)ₙPhNH-, PhNH- und Ph(CH₂)ₙNH-ausgewählt ist, wobei Ph für eine Phenylgruppe und n für eine ganze Zahl steht, vorzugsweise wobei n eine von 1, 2, 3, 4, 5 oder 6 ist.

10. Proteinbindendes Produkt nach einem der vorhergehenden Ansprüche, wobei es sich bei den porösen Polymerkügelchen um Agarose- oder vernetzte Agarosekügelchen, Hydroxymethacrylatkügelchen oder andere poröse Kunststoffkügelchen handelt.

11. Verwendung des proteinbindenden Produkts nach einem der Ansprüche 1-10 zur extrakorporalen Senkung der Spiegel des mindestens einen Galectins, bevorzugt Galectin-1, Galectin-3 und Galectin-8, und der Spiegel des mindestens einen Antikörpers, bevorzugt eines Blutgruppe-A-spezifischen Antikörpers, eines Blutgruppe-B-spezifischen Antikörpers, eines Autoantikörpers, bevorzugter eines AChR(Acetylcholin-Rezeptor)-Antikörpers und eines MuSK(muskelspezifische Kinase)-Rezeptor-Antikörpers, in menschlichem Blutplasma.

12. Vorrichtung, enthaltend das proteinbindende Produkt nach einem der Ansprüche 1-10, wobei es sich bei der Vorrichtung um eine Säule oder einen Schlauch handelt.

13. Verfahren zur extrakorporalen Senkung des Spiegels mindestens eines Galectins und mindestens eines Antikörpers in menschlichem Blutplasma, wobei das Verfahren die folgenden Schritte umfasst:
a) Leiten des Blutplasmas durch die Vorrichtung nach Anspruch 12,
b) Bindenlassen des mindestens einen Galactins und des mindestens einen Antikörpers, bevorzugt eines Blutgruppe-A-spezifischen Antikörpers, eines Blutgruppe-B-spezifischen Antikörpers, eines Autoantikörpers, bevorzugter eines AChR(Acetylcholin-Rezeptor)-Antikörpers und eines MuSK(muskelspezifische Kinase)-Rezeptor-Antikörpers, an den mindestens einen Antikörperbindungsliganden,
c) Fließenlassen mindestens eines Blutplasmavolumens durch die Vorrichtung nach dem Fließen eines Blutplasmavolumens durch die Vorrichtung,
d) Wiederholen von Schritt c), bis die gewünschte Senkung des Spiegels des mindestens einen Galectins und des mindestens einen Antikörpers im Blutplasma erreicht ist.

14. Verfahren nach Anspruch 13, wobei
- das Derivat von Galβ1-4GlcNAcβ-O- oder ein Derivat davon nach Anspruch 1 als Ligand verwendet wird, wenn der Galectin-3-Spiegel gesenkt werden soll.

15. Verfahren nach einem der Ansprüche 13 und 14, wobei das Verfahren zusätzlich mindestens einen der folgenden Schritte umfasst:
Messen der Spiegel des mindestens einen Galectins und/oder des mindestens einen Antikörpers während der Behandlung und
Berechnen der Senkung der Spiegel von Galactose bindenden Proteinen und/oder mindestens einem anderen Protein.

## Revendications

1. Produit liant une protéine comprenant une ou plusieurs billes de polymère poreuses, dans lequel au moins un ligand est lié aux surfaces desdites billes de polymère par l'intermédiaire d'un espaceur (R), et
dans lequel l'au moins un ligand est un dérivé de Galβ1 -3GlcNAcβ-O-,
dans lequel le groupe 3-OH de Gal est substitué par l'un des groupes suivants :
a) un groupe 3-OR2 où le groupe R2 contient un groupe alkyle ou un groupe aromatique,
b) un groupe 3-OR2 où le groupe R2 contient un groupe D-galactosyle ou un groupe GalNAc,
c) un groupe 3-NH-CO-R2 où le groupe R2 contient un groupe alkyle ou un groupe aromatique,
d) un groupe 3,5-diméthoxybenzamido, et/ou dans lequel ledit au moins un ligand est Galβ1-3GalNAcβ-O-et/ou un dérivé de celui-ci, dans lequel ledit dérivé dudit au moins un ligand a une structure dans laquelle le groupe 3-OH de Gal est substitué par l'un des groupes a)à d) ci-dessus, et/ou
dans lequel l'au moins un ligand est un dérivé de Galβ1-4GlcNAcp-O-,
dans lequel le groupe 3-OH de Gal est substitué par l'un des groupes a) à d) ci-dessus, à l'exception de b) où le groupe R2 contient un groupe D-galactosyle,
dans lequel ledit au moins un ligand a la capacité de se lier à au moins une galectine dans le sang humain, et dans lequel ledit produit liant une protéine comprend en outre des billes de polymère ayant au moins un composé ou ligand lié de manière covalente ayant la capacité de se lier à au moins un anticorps dans le sang humain.

2. Produit liant une protéine selon la revendication 1, dans lequel ledit au moins un anticorps est un anticorps spécifique du groupe sanguin A, un anticorps spécifique du groupe sanguin B ou un auto-anticorps.

3. Produit liant une protéine selon la revendication 2, dans lequel ledit auto-anticorps est un anticorps anAChR (récepteur de l'acétylcholine) ou un anticorps de récepteur Musk (kinase spécifique d'un muscle).

4. Produit liant une protéine selon l'une quelconque des revendications précédentes, dans lequel ledit au moins un ligand ayant la capacité de se lier spécifiquement à des galectines dans le plasma sanguin humain est de préférence la galectine-1, la galectine-3 et la galectine-8.

5. Produit liant une protéine selon l'une quelconque des revendications 1 et 2, dans lequel ledit ligand ayant la capacité de se lier à au moins un anticorps est un antigène du groupe sanguin A et/ou B.

6. Produit liant une protéine selon l'une quelconque des revendications précédentes, dans lequel la concentration du ligand est dans une plage de 0,1 à 20 µmol par mL de polymère, de préférence de 5 à 20 µmol par mL de polymère.

7. Produit liant une protéine selon l'une quelconque des revendications précédentes, dans lequel la concentration du au moins un ligand est dans une plage de 0,1 à 5 µmol par mL de polymère.

8. Produit liant une protéine selon l'une quelconque des revendications précédentes, dans lequel l'espaceur (R) est lié de manière covalente aux surfaces desdites billes de polymère poreuses et est lié de manière glycosidique au ligand.

9. Produit liant une protéine selon l'une quelconque des revendications précédentes, dans lequel l'espaceur est choisi dans le groupe constitué de (CH₂)ₙNH-, (CH₂)ₙPhNH-, PHNH- et Ph(CH₂)ₙNH-, dans lequel Ph est un groupe phényle et n est un nombre entier, de préférence dans lequel n est l'un de 1, 2, 3, 4, 5 ou 6.

10. Produit liant une protéine selon l'une quelconque des revendications précédentes, dans lequel lesdites billes de polymère poreuses sont des billes d'agarose ou d'agarose réticulé, des billes d'hydroxyméthacrylate ou d'autres billes de plastique poreuses.

11. Utilisation dudit produit liant une protéine selon l'une quelconque des revendications 1 à 10 pour la réduction extracorporelle des taux de ladite au moins une galectine, de préférence galectine-1, galectine-3 et galectine-8 ; et des taux dudit au moins un anticorps, de préférence un anticorps spécifique du groupe sanguin A, un anticorps spécifique du groupe sanguin B, un auto-anticorps, plus préférablement un anticorps AChR (récepteur de l'acétylcholine) et un anticorps de récepteur Musk (kinase spécifique d'un muscle), dans le plasma sanguin humain.

12. Dispositif contenant ledit produit liant une protéine selon l'une quelconque des revendications 1 à 10, dans lequel ledit dispositif est une colonne ou un tube.

13. Procédé de réduction extracorporelle du taux d'au moins une galectine et d'au moins un anticorps dans le plasma sanguin humain, ledit procédé comprenant les étapes consistant à :
a) faire passer le plasma sanguin à travers le dispositif selon la revendication 12,
b) permettre à ladite galectine et audit anticorps, de préférence un anticorps spécifique du groupe sanguin A, un anticorps spécifique du groupe sanguin B, un auto-anticorps, plus préférablement un anticorps AChR (récepteur de l'acétylcholine) et un anticorps de récepteur Musk (kinase spécifique d'un muscle) de se lier audit ligand liant un anticorps,
c) laisser passer au moins un volume de plasma sanguin à travers le dispositif après le passage d'un volume de plasma sanguin à travers le dispositif,
d) répéter l'étape c) jusqu'à ce que la réduction souhaitée du taux de ladite au moins une galectine et dudit au moins un anticorps dans le plasma sanguin soit atteinte.

14. Procédé selon l'une quelconque des revendications 13, dans lequel
- le dérivé de Galβ1 -4GlcNAcβ-O- ou un dérivé de celui-ci selon la revendication 1 est utilisé comme ligand lorsque le taux de galectine-3 doit être réduit.

15. Procédé selon l'une quelconque des revendications 13 et 14, dans lequel ledit procédé comprend en outre au moins l'une des étapes suivantes :
mesure des taux de ladite au moins une galectine et/ou dudit au moins un anticorps pendant le traitement et
calcul de la réduction des taux de protéines liant le galactose et/ou d'au moins une autre protéine.
